# EUROPEAN PATENT APPLICATION

(11) **EP 3 741 364 A1**
(43) Date of publication of application: **25.11.2020**
(21) Application number: 19741485.7
(22) Date of filing: 16.01.2019
(51) Int. Cl.: A61K 31/167, A61K 31/245, A61K 31/445, A61K 45/00, A61K 47/32, A61P 23/02

(54) **PHARMACEUTICAL COMPOSITION, PATCH AND MANUFACTURING METHOD THEREOF, ANALGESIC METHOD, AND APPLICATION**

(30) Priority: 17.01.2018 CN 201810045015
(71) Applicant: Zhang, Jie, Shanghai 200031 (CN)
(72) Inventor: Zhang, Jie, Shanghai 200031 (CN)
(74) Representative: Isarpatent
(86) International application number: PCT/CN2019/071898
(87) International publication number: WO 2019/141178

(57) **Abstract**

The present invention provides a pharmaceutical composition, a patch and a manufacturing method thereof, an analgesic method, and an application. The pharmaceutical composition comprises a single anesthetic active ingredient. The anesthetic active ingredient comprises a water-dissolving anesthetic active ingredient and an anesthetic active ingredient existing in an insoluble form. The mass percent content of the anesthetic active ingredient in the pharmaceutical composition is 2% or more. The mass percent content of the water-dissolving anesthetic active ingredient in the pharmaceutical composition is 1% or less. The anesthetic active ingredient comprises lidocaine, tetracaine, bupivacaine, articaine, cinchocaine, dibucaine, etidocaine, levobupivacaine, mepivacaine, prilocaine, ropivacaine, trimecaine, benzocaine or procaine. The pharmaceutical composition and the patch can provide continuous analgesia for 12-30 hours. The patch is resistant to a certain amount of external squeezing force, preventing a formulation layer covered by the same from being squeezed out, thereby maintaining the thickness of the formulation layer.

## Description

The present application claims the priority of Chinese patent application CN 201810045015.8 filed on January 17, 2018. The aforementioned Chinese patent application is incorporated into the present application by reference in its entirety.

### Technical Field

The present invention relates to a pharmaceutical composition, a patch and a manufacturing method thereof, an analgesic method, and an application.

### Background Arts

Local anesthetic preparations are used for preventing or treating pains in skin or mucosa, for example: 2% lidocaine hydrochloride gel is used for alleviating the sunburn-related pains; EMLA, an emulsion that contains lidocaine and prilocaine in a form of low co-melting point oil drops, is used for the anesthesia of intact skin before an operation accompanied with pains; Lidoderm, a curing gel patch containing 5% lidocaine, is used for relieving postherpetic neuralgia (the skin is intact *per se*); Pliaglis, an emulsion that contains 7% lidocaine and 7% tetracaine in a form of low co-melting point oil drops, is used for the anesthesia of intact skin before an operation accompanied with pains. However, the aforementioned local anesthetics have their respective serious limitations. For example: the lidocaine hydrochloride gel cannot be used for the anesthesia of intact skin; the prilocaine contained in EMLA may cause methemoglobinemia; the tetracaine contained in Pliaglis may cause the allergic reactions related to local ester anesthetics, and the tetracaine will be hydrolyzed. Therefore, Pliaglis must be kept in cold storage, in order to reduce the rate of hydrolysis. Lidoderm cannot anesthetize the intact skin in 120 minutes, or even 4 hours, and Lidoderm should not be used for more than 12 hours at a time. However, the common limitation of the aforementioned local anesthetic preparations is that they cannot achieve long-term continuous and safe analgesia.

In the treatment of many diseases, an ideal product should achieve continuous analgesia for 12, 18, 24, and even 30 hours. Therefore, currently there is an urgent need to seek a pharmaceutical composition with long-term continuous and safe analgesia.

In addition, the thickness of the formulation layer covering the affected part is very important, in order to achieve the long-term analgesia of the pharmaceutical composition on the affected part. If the formulation layer is too thin, the local anesthetic contained in the formulation layer will be absorbed too quick to provide an expected analgesia time. In the prior art, the formulation layer is generally applied onto the affected part, and then covered with a medical film. During analgesia, the formulation layer is squeezed by external force, so the formulation layer at the affected part is thinned, which further reduces the effective analgesia time greatly. Therefore, currently there is also an urgent need to seek a patch that can ensure the thickness of a formulation layer.

### Content of the Present Invention

The technical problem to be solved in the present invention is to provide a pharmaceutical composition, a patch, a manufacturing method thereof, an analgesic method and an application, in order to overcome the defect of the common limitation that the local anesthetic preparations in the prior art cannot achieve a long-term continuous and safe analgesia. The pharmaceutical composition and the patch can provide continuous and safe analgesia for 12, 18, 24, and even 30 hours, and the patch is resistant to a certain amount of external squeezing force, preventing a formulation layer covered by the same from being squeezed out, thereby maintaining the thickness of the formulation layer.

The present invention solves the above-mentioned technical problem through the following technical solutions:
The present invention provides a pharmaceutical composition. The pharmaceutical composition comprises a single anesthetic active ingredient. The single anesthetic active ingredient comprises a part of the water-dissolving anesthetic active ingredient dissolved in water and another part anesthetic active ingredient existing in an undissolved form. The mass percent content of the anesthetic active ingredient in the pharmaceutical composition is 2% or more. The mass percent content of the water-dissolved anesthetic active ingredient in the pharmaceutical composition is 1% or less. The anesthetic active ingredient comprises lidocaine, tetracaine, bupivacaine, articaine, cinchocaine, dibucaine, etidocaine, levobupivacaine, mepivacaine, prilocaine, ropivacaine, trimecaine, benzocaine or procaine.

The pharmaceutical composition in the present invention can achieve continuous analgesia for 12, 18, 24, and even 30 hours (for example: providing analgesia for more than 24 h for a patient with a postoperative incision pain at the incision wound; providing analgesia for more than 12 or 24 hours for a patient with postherpetic neuralgia in the pain point). The preparation (the "preparation" in the present invention refers to the pharmaceutical composition) can be used for easing the pain in the herpes period of herpes zoster, postherpetic neuralgia, neuroma pain, pain phantom limb, diabetic peripheral neuropathic pain, arthralgia, osteoarthritis pain, back pain, pain caused by gout, pain caused by soft tissue injury, postoperative incision pain, burn pain or pain caused by removal of burn scabs.

Through comparison with the pharmaceutical composition in the present invention and the pharmaceutical compositions (the products containing two or more local anesthetic ingredients, like EMLA) on the market, lidocaine, as the preparation of single anesthetic active ingredient, has better advantages: (1) this preparation is safer, easy to be approved, and can be used for a long term (continuous use for more than 3 months), specifically: CD lidocaine is the most commonly used local anesthetic, with a generally known safety; ② this preparation is easier to be approved by the relevant government authorities (like NMPA and FDA) for use in the market; ③ lidocaine is the only local anesthetic approved by US FDA for long-term use (typically defined as more than three months) (for many diseases needing long-term analgesia, the approval for long-term use is very important, for example: in the analgesic application of postherpetic neuralgia, the patients with postherpetic neuralgia may need to use the analgesics for many years, if the local anesthetic contained in a preparation is not lidocaine, such preparation will be approved for long-term use only after lots of time-consuming and expensive tests like long-term toxicity test); (2) this preparation is able to anesthetize the intact skin in 120 minutes and even 90 minutes, specifically, it is also very important to have the ability to anesthetize the intact skin in 90 minutes or 120 minutes, for this is very important to timely relieve the patients from the pains, particularly, the pain like postherpetic neuralgia; and (3) this preparation also can be used for the analgesia of non-intact skin (for example, analgesia for the postoperative incision wound and the pain in the herpes period of herpes zoster).

In the present invention, the mass percent content of the anesthetic active ingredient in the pharmaceutical composition also may be more than 3%, more than 4%, more than 5%, more than 8% or more than 10%, for example, 3%-15%, 4%-10% or 5%-8%.

In the present invention, the mass percent content of the water-dissolved anesthetic active ingredient in the pharmaceutical composition also may be less than 0.7%, 0.5%-0.7% or less than 0.5%.

In the present invention, the particles of the anesthetic active ingredient may be the particles of anesthetic active ingredient conventionally used in the field, preferably the single particle weight of more than 80% of the particles of anesthetic active ingredient is 0.01 mg to 10 mg.

In the present invention, the pharmaceutical composition preferably includes a thickener, and the addition of thickener can increase the viscosity of preparation and prevent the preparation applied onto the skin from setting apart from the target. The thickener is preferably one or more of starch, carbomer and cellulose, and more preferably one or more of starch, hydroxyethyl cellulose and hydroxypropyl cellulose. The mass percent content of the thickener in the pharmaceutical composition is preferably 0.02%-4%, more preferably 0.02%-2%, and further more preferably 0.02%-1%.

In the present invention, the pharmaceutical composition preferably includes glycerin and/or propylene glycol, and the addition of glycerin and/or propylene glycol can reduce the risk of skin irritation. The mass percent content of the glycerin and/or propylene glycol in the pharmaceutical composition is preferably 2%-25%.

In the present invention, the pharmaceutical composition preferably includes a suspension agent, which can be the suspension agent commonly used in the field. The suspension agent is preferably carbomer, the strength of the suspension agent is preferably Pemulen TR-2, and the manufacturer of the suspension agent is Lubrizol. The mass percent content of the suspension agent in the pharmaceutical composition is preferably 0.10%-0.50%.

In the present invention, the pH value of the pharmaceutical composition may be more than 7 or more than 8, for example, 7-13, 7-11 or 7.5-9.5. A pH value regulator commonly used in the field can be used to regulate the pH value of the pharmaceutical composition to be the range of pH value above, for example: One or more of sodium tripolyphosphate, sodium bicarbonate, sodium hydroxide and potassium hydroxide can be used; Preferably, sodium hydroxide and/or potassium hydroxide is used. More preferably, sodium hydroxide is used. Further more preferably, when Pemulen TR-2 is used as the suspension agent in the pharmaceutical composition, the mass ratio between the sodium hydroxide and the Pemulen TR-2 is more than 9:16.

In the present invention, when the anesthetic active ingredient is tetracaine, preferably the mass percent content of tetracaine in the pharmaceutical composition is more than 0.5%, and the mass percent content of water-dissolved tetracaine in the pharmaceutical composition is less than 0.1%.

The present invention also provides a manufacturing method of the pharmaceutical composition, and the manufacturing method comprises the following step of: mixing the different ingredients of the pharmaceutical composition.

Preferably, the manufacturing method comprises the following steps of: (1) mixing other ingredients other than the anesthetic active ingredient in the pharmaceutical composition, and obtaining a mixed solution; (2) heating the mixed solution and the anesthetic active ingredient, and then cooling to the room temperature.

In the step (2), the heating temperature is preferably 75°C-85°C, for example, 80°C.

In the step (2), the heating is conducted preferably with stirring.

The present invention also provides a patch which comprises a cover film and the aforesaid pharmaceutical composition. The contact surface of the cover film to the skin is provided with at least one indentation. The opening area of the indentation accounts for more than 70% (100% excluded) of the total area of the contact surface, and the indentation is filled with the pharmaceutical composition.

In the present invention, the cover film may be made of a variety of materials or a combination of materials, and the materials used are generally soft and uneasy to be dented, selectively, foamed plastics, like polyurethane.

In the above-mentioned technical solution, the cover film has certain structure and hardness, and the cover film also has a structure of indentation. When the outside of the cover film is subject to pressure, the formulation in the structure of indentation is not easily squeezed out of the affected area.

In the present invention, the moisture vapor transmission rate of the cover film is preferably 100-10,000 g/m²/24 h, and more preferably 400-4,000 g/m²/24 h. The moisture vapor transmission rate (MVTR) is measured according to the universal methods in the industry. The materials and the MVTR within the limits set above can help to realize the normal breathing of the skin in the affected part better.

In the present invention, the thickness of the cover film is preferably 1-20 millimeters, and more preferably 3-10 millimeters.

In the present invention, the length of the cover film is preferably 3-50 centimeters, and more preferably 8-30 centimeters.

In the present invention, the area of the cover film is preferably 5-1,000 square centimeters, and more preferably 10-500 square centimeters.

In the present invention, the indentation is preferably configured as one or more of a cuboid, a cylinder and a hemispheroid. More preferably, the axis of the cylinder is perpendicular to the contact surface, and the axis of the hemispheroid is perpendicular to the contact surface. Further more preferably, the indentation is configured as a hemispheroid, and the opening of each indentation is tangent to the opening of the adjacent indentation.

In the present invention, the depth of the indentation may be less than 10 millimeters, preferably 0.2-10 millimeters, and more preferably 0.5-3 millimeters.

In the present invention, the cover film may be provided with only one indentation, the indentation has an indentation cavity, and the opening width of the indentation is preferably 2-40 millimeters, and more preferably 3-20 millimeters; the opening length of the indentation is preferably 2-50 centimeters, and more preferably 5-20 centimeters.

In the present invention, the cover film may also be provided with two or more indentations, and the number of indentations per square centimeter of the cover film is preferably 0.2-100, and more preferably 0.5-20; the spacing between the two neighboring indentations is preferably 0-5 millimeters, and more preferably 0-2 millimeters. Further more preferably, the two neighboring indentations are not connected.

In the present invention, the cover film is provided with only one indentation, the indentation has an indentation cavity, the contact surface of the indentation cavity to the skin is provided with several first through-holes, one or more layers of drug storage cavity units are provided in the indentation cavity, each layer of the drug storage cavity units comprises several drug storage micro-cavities, each drug storage micro-capacity is provided with a second through-hole on the side wall, and the second through-hole is used for communication of the two neighboring drug storage micro-cavities; the opening area of the first through-hole accounts for more than 70% (100% excluded) of the total area of the first surface;
wherein, preferably, all the drug storage micro-cavities form a loofah-like network structure together;
wherein, preferably, the drug storage micro-cavity is a regular-prism-shaped cavity, and the drug storage micro-cavities are arranged in a honeycomb structure;
wherein, preferably, the drug storage micro-cavities of the two neighboring layers are also in a staggered arrangement;
wherein, preferably, other surfaces of the indentation cavity except for the first surface are all of a sealed structure.

In the present invention, the depth of the indentation is preferably equal to the thickness of the cover film.

One side of the indentation away from the opening may be partially attached with a barrier film.

One side of the indentation away from the opening may be totally attached with a barrier film. When the side of the indentation away from the opening is totally attached with a barrier film, the formed covering film is a covering combination.

In the above-mentioned technical solution, the depth of the indentation is equal to the thickness of the covering film, so as to facilitate the processing and manufacturing. However, for some formulations, it is necessary to retain the moisture in the formulation layer that has applied onto the skin, wound or mucosa during use. One of the methods for retaining the moisture is to attach a layer of barrier film, such as a plastic film or a polyurethane film, to the side of the indentation away from the opening so as to form a covering combination, and then cover the formulation layer with this covering combination. If the formulation layer is applied onto a wound from which body fluid is exuded, the side of the indentation away from the opening may not be attached with a barrier film or partially attached with a barrier film.

In the present invention, preferably the indentation have the same structure, and the indentations are arranged evenly or in an array.

In the present invention, the indentation of the cover film is filled with the pharmaceutical composition, the opening is in direct contact with the affected part, and in this case, the area of the opening is the area of direct contact between the pharmaceutical composition and the affected part.

In the present invention, the outer edge of the opening may be a continuous closed curve, a number of tangential closed curves, a number of closed curves with only one intersection, or a number of closed curves without intersection.

In the present invention, the spacing refers to the minimum distance between the outer edges of two neighboring openings.

The present invention also provides an application of the pharmaceutical composition in the manufacturing of a drug for treating the pain in the herpes period of herpes zoster, postherpetic neuralgia, neuroma pain, phantom limb pain, diabetic peripheral neuropathic pain, arthralgia, osteoarthritis pain, back pain, pain caused by gout, pain caused by soft tissue injury, postoperative incision pain, burn pain or pain caused by removal of burn scabs.

The present invention also provides an application of the patch in the manufacturing of a medical device for treating the pain in the herpes period of herpes zoster, postherpetic neuralgia, neuroma pain, pain phantom limb, diabetic peripheral neuropathic pain, arthralgia, osteoarthritis pain, back pain, pain caused by gout, pain caused by soft tissue injury, postoperative incision pain, burn pain or pain caused by removal of burn scabs.

The present invention also provides an analgesic method by using the pharmaceutical composition, and the analgesic method comprises the following steps of: applying the pharmaceutical composition to the affected skin, and then covering the pharmaceutical composition with the barrier film.

In the present invention, the affected skin, for example, may be the skin suffering from pain in the herpes period of herpes zoster, the skin suffering from postherpetic neuralgia, the skin suffering from neuroma pain, the skin related to phantom limb pain, the skin suffering from diabetic peripheral neuropathic pain, the skin over a tissue suffering from arthralgia, the skin over a tissue suffering from osteoarthritis pain, the skin over back pain, the skin over a tissue suffering from pain caused by gout, the skin over a tissue suffering from pain caused by soft tissue injury, the skin suffering from postoperative incision pain, the skin suffering from burn pain, or the skin suffering from pain caused by removal of burn scabs.

In the present invention, preferably, the pharmaceutical composition is maintained on the affected skin for more than 8, 12, 18, 24, or 30 hours.

In the present invention, the thickness of the pharmaceutical composition on the affected skin is preferably not less than 0.5, 1 or 2 millimeters.

The present invention also provides an analgesic method by using the patch, and the analgesic method comprises the following step of: bringing the opening of the indentation in the patch into contact with the affected skin.

In the present invention, the affected skin, for example, may be the skin suffering from pain in the herpes period of herpes zoster, the skin suffering from postherpetic neuralgia, the skin suffering from neuroma pain, the skin related to phantom limb pain, the skin suffering from diabetic peripheral neuropathic pain, the skin over a tissue suffering from arthralgia, the skin over a tissue suffering from osteoarthritis pain, the skin over back pain, the skin over a tissue suffering from pain caused by gout, the skin over a tissue suffering from pain caused by soft tissue injury, the skin suffering from postoperative incision pain, the skin suffering from burn pain, or the skin suffering from pain caused by removal of burn scabs.

In the present invention, preferably, the pharmaceutical composition is maintained on the affected skin for more than 8, 12, 18, 24, or 30 hours.

In the present invention, the thickness of the pharmaceutical composition contained in the patch on the affected skin is preferably not less than 0.5, 1 or 2 millimeters.

In the present invention, intact skin or intact normal human skin refers to the skin with an intact main barrier (cuticle) to foreign matters.

In the present invention, unless otherwise stipulated, the percentage refers to the mass percent content.

In the present invention, the expressions like "able to anesthetize the intact normal human skin within 120 min" refer to that, after application of the pharmaceutical composition at normal room temperature, most people's skin will be anesthetized in that time, and medically speaking, those of skill in the art should understand that, this expression doesn't mean that everyone's skin will be anesthetized in that time.

On the basis of meeting common knowledge in the art, the above-mentioned various preferred conditions can be combined in any form, such that various preferred examples of the present invention are obtained.

The reagents and raw materials used in the present invention are commercially available.

The present invention has the following positive improvement effects: The present invention provides a pharmaceutical composition, a patch and a manufacturing method thereof, an analgesic method, and an application. The preparation and the patch can provide continuous and safe analgesia for 12, 18, 24, and even 30 hours, and the patch is resistant to a certain amount of external squeezing force, preventing a formulation layer covered by the same from being squeezed out, thereby maintaining the thickness of the formulation layer. The pharmaceutical composition can be used for easing the pain in the herpes period of herpes zoster, postherpetic neuralgia, neuroma pain, phantom limb pain, diabetic peripheral neuropathic pain, arthralgia, osteoarthritis pain, back pain, pain caused by gout, pain caused by soft tissue injury, postoperative incision pain, burn pain or pain caused by removal of burn scabs.

Compared with the pharmaceutical composition in the present invention and the pharmaceutical compositions (the products containing two or more local anesthetic ingredients, for example, EMLA) in the market, lidocaine, as the preparation of single anesthetic active ingredient, has better advantages: (1) this preparation is safer, easy to be approved, and can be used for a long term (continuous use for more than three months), (2) this preparation is able to anesthetize the intact skin within 120 minutes and even 90 minutes; and (3) this preparation also can be used for the analgesia of non-intact skin.

### Brief Description of the Drawings

Figure 1 is the structure diagram of the cover film and the barrier film of the patch as defined in Embodiment 27.
Figure 2 is the structure diagram of the cover combination of the patch as defined in Embodiment 27.
Figure 3 is the structure diagram of the cover film and the barrier film of the patch as defined in Embodiment 28, Embodiments 32 to 36.
Figure 4 is the structure diagram of the covering combination of the patch as defined in Embodiment 28, Embodiments 32 to 36.
Figure 5 is the structure diagram of the cover film and the barrier film of the patch as defined in Embodiments 29 to 31.
Figure 6 is the structure diagram of the cover combination of the patch as defined in Embodiments 29 to 31.
Figure 7 is the structure diagram of the cover film and the barrier film of the patch as defined in Embodiments 37 to 41.
Figure 8 is the structure diagram of the cover combination of the patch as defined in Embodiments 37 to 41.
Figure 9 is the structure diagram of the cover film and the barrier film of the patch as defined in Embodiment 42.
Figure 10 is the structure diagram of the cover combination of the patch as defined in Embodiment 42.
Figure 11 is the structure diagram of the cover film of the patch as defined in Embodiments 43 to 46.
Figure 12 is the structure diagram of the cover combination of the patch as defined in Embodiments 43 to 46.

Description of the signs in the drawings:
Indentation 1
Barrier film 2
Bonding layer 3

### Detailed Description of the Preferred Embodiment

The following examples further illustrate the present invention, but the present invention is not limited thereto. Experimental methods with specific conditions are not indicated in the following examples, but can be chosen according to conventional methods and conditions or commodity instructions.

In Embodiments 1 to 24, among the particles of the anesthetic active ingredient, the single particle weight of more than 80% of the particles of anesthetic active ingredient is 0.01-10 mg.

### Embodiment 1

A pharmaceutical composition, which was composed of the following ingredients:

| Ingredient | Mass percent content/% |
|---|---|
| Lidocaine | 4 |
| Pemulen TR-2 | 0.24 |
| Sodium hydroxide | 0.13 |
| Water | Made up to 100 |

The manufacturing method of the pharmaceutical composition is as follows:
adding the sodium hydroxide and the suspension agent carbomer Pemulen TR-2 into the water, and mixing until a uniformly viscous liquid was obtained; and then, adding lidocaine crystals, heating to 80°C, mixing quickly, and cooling to the room temperature, so as to obtain the pharmaceutical composition.

Effect data: The obtained pharmaceutical composition is recorded as Formulation A, and the obtained pharmaceutical composition was a viscous solution containing dissolved lidocaine and undissolved lidocaine, wherein undissolved lidocaine exists in the viscous solution in the form of crystal suspension; the pH value of the obtained pharmaceutical composition was higher than 8; lidocaine in 1 ml of the solution was 40 mg, wherein: about 5 mg was dissolved lidocaine, and about 35 mg was undissolved and suspended lidocaine crystals.

### Embodiment 2

A pharmaceutical composition, which was composed of the following ingredients:

| Ingredient | Mass percent content/% |
|---|---|
| Lidocaine | 5 |
| Pemulen TR-2 | 0.24 |
| Sodium hydroxide | 0.13 |
| Starch | 0.3 |
| Water | Made up to 100 |

The manufacturing method of the pharmaceutical composition is as follows:
adding the sodium hydroxide and the suspension agent carbomer Pemulen TR-2 into the water, and mixing until a uniformly viscous liquid was obtained; and then, adding lidocaine crystals and starch, heating to 80°C, mixing quickly, and cooling to the room temperature, so as to obtain the pharmaceutical composition.

Effect data: The obtained pharmaceutical composition is recorded as Formulation B, and the obtained pharmaceutical composition was a viscous solution containing the dissolved lidocaine and the undissolved lidocaine, wherein the undissolved lidocaine exists in the viscous solution in the form of crystal suspension; the pH value of the obtained pharmaceutical composition was higher than 8; the lidocaine in 1 ml of solution was 50 mg, wherein: about 5 mg was dissolved lidocaine, and about 45 mg was undissolved and suspended lidocaine crystal.

### Embodiment 3

A pharmaceutical composition, which is composed of the following ingredients:

| Ingredient | Mass percent content/% |
|---|---|
| Lidocaine | 5 |
| Pemulen TR-2 | 0.25 |
| Sodium hydroxide | 0.14 |
| Starch | 0.3 |
| Glycerin | 15 |
| Water | Made up to 100 |

The manufacturing method of the pharmaceutical composition was as follows:
adding the sodium hydroxide and the suspension agent carbomer Pemulen TR-2 into the water, and mixing until a uniformly viscous liquid was obtained; and then, adding lidocaine crystal, starch and glycerin, heating to 80°C, mixing quickly, and cooling to the room temperature, so as to obtain the pharmaceutical composition.

Effect data: The obtained pharmaceutical composition was recorded as Formulation C, and the obtained pharmaceutical composition was a viscous solution containing dissolved lidocaine and undissolved lidocaine, wherein the undissolved lidocaine exists in the viscous solution in the form of crystal suspension; the pH value of the obtained pharmaceutical composition was higher than 8; most lidocaine existed in the form of undissolved crystal suspension.

### Embodiment 4

A pharmaceutical composition, which is composed of the following ingredients:

| Ingredient | Mass percent content/% |
|---|---|
| Lidocaine | 10 |
| Pemulen TR-2 | 0.24 |
| Sodium hydroxide | 0.13 |
| Starch | 0.3 |
| Water | Made up to 100 |

The manufacturing method of the pharmaceutical composition was the same as that in Embodiment 2.

Effect data: The obtained pharmaceutical composition was recorded as Formulation D, and the obtained pharmaceutical composition was a viscous solution containing dissolved lidocaine and undissolved lidocaine, wherein the undissolved lidocaine existed in the viscous solution in the form of crystal suspension; the pH value of the obtained pharmaceutical composition was higher than 8; the lidocaine in 1 ml of the solution was 100 mg, wherein: about 5 mg was dissolved lidocaine, and about 95 mg was the undissolved and suspended lidocaine crystals.

### Embodiment 5

A pharmaceutical composition, which was composed of the following ingredients:

| Ingredient | Mass percent content/% |
|---|---|
| Lidocaine | 15 |
| Pemulen TR-2 | 0.24 |
| Sodium hydroxide | 0.13 |
| Starch | 0.3 |
| Water | Made up to 100 |

The manufacturing method of the pharmaceutical composition was the same as that in Embodiment 2.

Effect data: The obtained pharmaceutical composition was recorded as Formulation E, and the obtained pharmaceutical composition was a viscous solution containing dissolved lidocaine and undissolved lidocaine, wherein the undissolved lidocaine existed in the viscous solution in the form of crystal suspension; the pH value of the obtained pharmaceutical composition was higher than 8; the lidocaine in 1 ml of the solution was 150 mg, wherein: about 5 mg was dissolved lidocaine, and about 145 mg is the undissolved and suspended lidocaine crystals.

### Embodiment 6

A pharmaceutical composition, which is composed of the following ingredients:

| Ingredient | Mass percent content/% |
|---|---|
| Bupivacaine | 4 |
| Pemulen TR-2 | 0.25 |
| Sodium hydroxide | 0.16 |
| Starch | 0.4 |
| Water | Made up to 100 |

The manufacturing method of the pharmaceutical composition is as follows:
adding the sodium hydroxide and the suspension agent carbomer Pemulen TR-2 into the water, and mixing until a uniformly viscous liquid is obtained; and then, adding bupivacaine crystals and starch, heating to 80°C, mixing quickly, and cooling to the room temperature, so as to obtain the pharmaceutical composition.

Effect data: The obtained pharmaceutical composition is recorded as Formulation F, and only less than a quarter of the total amount of bupivacaine in the obtained pharmaceutical composition is dissolved, while the rest exists in the form of particles, i.e., the mass fraction of the dissolved bupivacaine is less than 1%, and the percentage is the percentage of the mass of the dissolved bupivacaine in the total mass of the obtained pharmaceutical composition.

### Embodiment 7

A pharmaceutical composition, which is composed of the following ingredients:

| Ingredient | Mass percent content/% |
|---|---|
| Tetracaine | 4 |
| Pemulen TR-2 | 0.25 |
| Sodium hydroxide | 0.16 |
| Starch | 0.4 |
| Water | Made up to 100 |

The manufacturing method of the pharmaceutical composition is as follows:
adding the sodium hydroxide and the suspension agent carbomer Pemulen TR-2 into the water, and mixing until a uniformly viscous liquid is obtained; and then, adding tetracaine crystal and starch, heating to 80°C, mixing quickly, and cooling to the room temperature, so as to obtain the pharmaceutical composition.

Effect data: The obtained pharmaceutical composition is recorded as Formulation G, and only less than one tenth of the total amount of tetracaine in the obtained pharmaceutical composition is dissolved, while the rest exists in the form of particles, i.e., the mass fraction of the dissolved tetracaine is less than 0.4%, and the percentage is the percentage of the mass of the dissolved tetracaine in the total mass of the obtained pharmaceutical composition.

### Embodiments 8 to 18

The anesthetic active ingredient as defined in embodiment 1 is replaced with articaine, cinchocaine, dibucaine, etidocaine, levobupivacaine, mepivacaine, prilocaine, ropivacaine, trimecaine, benzocaine and procaine respectively, so as to obtain Embodiments 8 to 18.

Effect data: The obtained pharmaceutical composition is a viscous solution containing dissolved form of the anesthetic active ingredient and undissolved form of the anesthetic active ingredient, wherein the undissolved anesthetic active ingredient exists in the viscous solution in the form of crystal suspension; the pH value of the obtained pharmaceutical composition is higher than 8; the anesthetic active ingredient in 1 ml of the solution is 40 mg, wherein: about 5 mg is dissolved, and about 35 mg is undissolved and suspended crystals.

### Embodiment 19

A pharmaceutical composition, which is composed of the following ingredients:

| Ingredient | Mass percent content/% |
|---|---|
| Lidocaine | 2 |
| Sodium hydroxide | 0.13 |
| Water | Made up to 100 |

The manufacturing method of the pharmaceutical composition is as follows:
adding sodium hydroxide into the water, mixing well, and then, adding lidocaine crystal, heating to 80°C, mixing quickly, and cooling to the room temperature, so as to obtain the pharmaceutical composition.

Effect data: the pH value of the obtained pharmaceutical composition is higher than 8; the lidocaine in 1 ml of the solution is 20 mg, wherein: about 5 mg is the dissolved lidocaine, and about 15 mg is undissolved and suspended lidocaine crystals.

### Embodiment 20

A pharmaceutical composition, which is composed of the following ingredients:

| Ingredient | Mass percent content/% |
|---|---|
| Lidocaine | 3 |
| Pemulen TR-2 | 0.24 |
| Sodium hydroxide | 0.13 |
| Water | Made up to 100 |

The manufacturing method is the same as that in Embodiment 1. The pH value of the obtained pharmaceutical composition is higher than 8; the lidocaine in 1 ml of the solution is 30 mg, wherein: about 5 mg is dissolved lidocaine, and about 25 mg is the undissolved and suspended lidocaine crystals.

### Embodiment 21

A pharmaceutical composition, which is composed of the following ingredients:

| Ingredient | Mass percent content/% |
|---|---|
| Lidocaine | 8 |
| Pemulen TR-2 | 0.24 |
| Sodium hydroxide | 0.13 |
| Starch | 0.02 |
| Glycerin | 2 |
| Water | Made up to 100 |

The manufacturing method is the same as that in Embodiment 3. The pH value of the obtained pharmaceutical composition is higher than 8; the lidocaine in 1 ml of the solution is 80 mg, wherein: about 5 mg is dissolved lidocaine, and about 75 mg is undissolved and suspended lidocaine crystals.

### Embodiment 22

A pharmaceutical composition, which is composed of the following ingredients:

| Ingredient | Mass percent content/% |
|---|---|
| Lidocaine | 15 |
| Pemulen TR-2 | 0.24 |
| Sodium hydroxide | 0.13 |
| Starch | 4 |
| Glycerin | 25 |
| Water | Made up to 100 |

The manufacturing method is the same as that in Embodiment 3. The pH value of the obtained pharmaceutical composition is higher than 8; the lidocaine in 1 ml of the solution is 150 mg, wherein: about 5 mg is dissolved lidocaine, and about 145 mg is undissolved and suspended lidocaine crystals.

### Embodiment 23

A pharmaceutical composition, which is composed of the following ingredients:

| Ingredient | Mass percent content/% |
|---|---|
| Lidocaine | 10 |
| Pemulen TR-2 | 0.24 |
| Sodium hydroxide | 0.14 |
| Starch | 1 |
| Water | Made up to 100 |

The manufacturing method is the same as that in Embodiment 2. The pH value of the obtained pharmaceutical composition is higher than 8; the lidocaine in 1 ml of the solution is 100 mg, wherein: about 7 mg is dissolved lidocaine, and about 93 mg is undissolved and suspended lidocaine crystals.

### Embodiment 24

A pharmaceutical composition, which is composed of the following ingredients:

| Ingredient | Mass percent content/% |
|---|---|
| Tetracaine | 0.5 |
| Pemulen TR-2 | 0.24 |
| Sodium hydroxide | 0.14 |
| Water | Made up to 100 |

The manufacturing method is the same as that in Embodiment 1, and the difference is that the anesthetic active ingredient is tetracaine. The pH value of the obtained pharmaceutical composition is higher than 8; the tetracaine in 1 ml of the solution is 5 mg, wherein: about 1 mg is dissolved tetracaine, and about 4 mg is undissolved and suspended tetracaine crystals.

### Embodiment 25

A pharmaceutical composition, which is composed of the following ingredients:

| Ingredient | Mass percent content/% |
|---|---|
| Lidocaine | 10 |
| Pemulen TR-2 | 0.1 |
| Sodium hydroxide | 0.06 |
| Water | Made up to 100 |

The manufacturing method is the same as that in Embodiment 1. The pH value of the obtained pharmaceutical composition is higher than 8; the lidocaine in 1 ml of the solution is 100 mg, wherein: about 7 mg is dissolved lidocaine, and about 93 mg is undissolved and suspended lidocaine crystals.

### Embodiment 26

A raw material composition of pharmaceutical composition, which is composed of the following ingredients:

| Ingredient | Mass percent content/% |
|---|---|
| Lidocaine | 10 |
| Pemulen TR-2 | 0.5 |
| Sodium hydroxide | 0.29 |
| Water | Made up to 100 |

The manufacturing method is the same as that in Embodiment 1. The pH value of the obtained pharmaceutical composition is higher than 8; the lidocaine in 1 ml of the solution is 100 mg, wherein: about 7 mg is dissolved lidocaine, and about 93 mg is undissolved and suspended lidocaine crystals.

### Embodiment 27

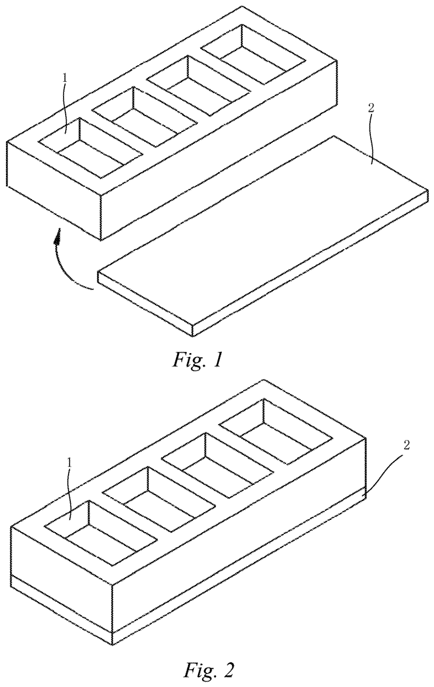

A patch, which comprises the cover film as shown in Figure 1 and Formulation D. The cover film is a polyurethane film, of which the moisture vapor transmission rate is 100 g/m²/24 h, thickness is 3 millimeters, length is 10 centimeters, and area is 15 square centimeters. The contact surface of the cover film to the skin is provided with several indentations 1 which are in rectangle shape and arranged in an array manner. The number of indentations per square centimeter of the covering film is 0.8, each indentation is 3 millimeters deep, the spacing between two neighboring indentations is 2 millimeters, and the opening area of the indentations accounts for 70% of the total area of the contact surface. One side of the indentation away from the opening is attached with a barrier film 2 so as to form the covering combination as shown in Figure 2. The barrier film 2 is a polyurethane film, of which the moisture vapor transmission rate is 100 g/m²/24 h. The formulation is filled in the indentations.

### Embodiments 28 to 31

The structural parameters of the covering film combination used in the patch as defined in Embodiments 28 to 31 are as follows, and others are the same as those in embodiment 27. The indentations are filled with Formulation D.

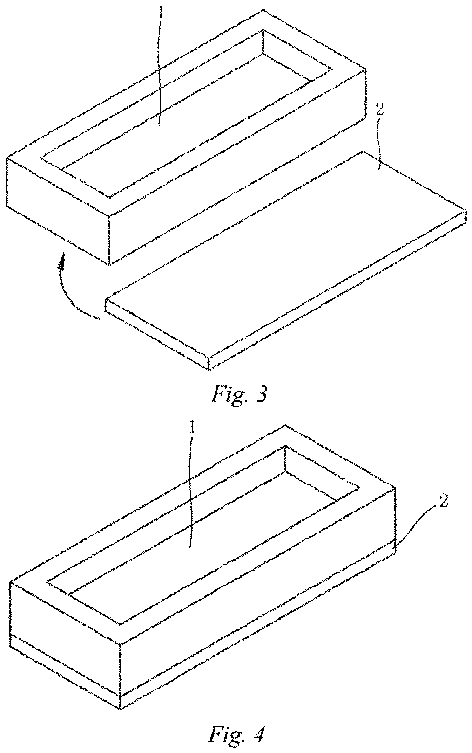

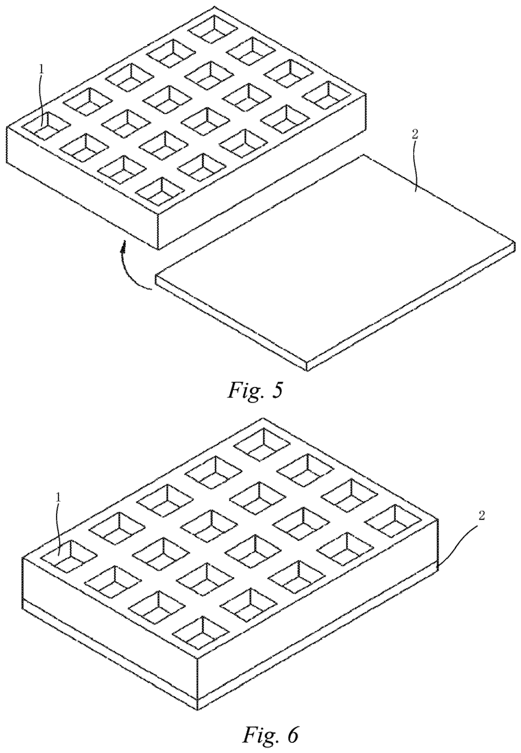

Wherein the structure diagram of the cover film and the barrier film of the patch as defined in Embodiment 28 is as shown in Figure 3, and the cover combination formed is as shown in Figure 4. Wherein the structure diagram of the covering film and the barrier film of the patch as defined in Embodiments 29 to 31 is as shown in Figure 5, and the covering combination formed is as shown in Figure 6.

| Index | | Embodiment 28 | Embodiment 29 | Embodiment 30 | Embodiment 31 |
|---|---|---|---|---|---|
| Cover film | MVTR (g/m²/24 h) | 400 | 4,000 | 7,000 | 10,000 |
| | Thickness/mm | 0.2 | 2 | 0.5 | 10 |
| | Length/cm | 3 | 30 | 8 | 50 |
| | Area/cm² | 5 | 500 | 10 | 1,000 |
| Indentation | Number of indentations per square centimeter | 0.2 | 20 | 0.5 | 100 |
| | Depth/mm | 0.2 | 2 | 0.5 | 10 |
| | Spacing between the two neighboring indentations/mm | / | 1 | 0.5 | 5 |
| The proportion of opening area in the total area of contact surface/% | | 70 | 75 | 80 | 95 |
| Barrier film | MVTR (g/m²/24 h) | 400 | 4,000 | 7,000 | 10,000 |

### Embodiment 32

A patch comprises the cover film as shown in Figure 3 and Formulation D. The cover film is a polyurethane film, of which the moisture vapor transmission rate is 100 g/m²/24 h. The contact surface of the cover film to the skin is provided with a indentation 1 which is in rectangle shape. The indentation 1 is 3 millimeters deep, the opening width of the indentation 1 is 15 millimeters, the opening length of the indentation 1 is 5 centimeters, and the opening area of the indentation 1 accounts for 70% of the total area of the contact surface. One side of the indentation 1 away from the opening is also attached with a barrier film 2 so as to form the covering combination as shown in Figure 4. The barrier film 2 is a polyurethane film, of which the moisture vapor transmission rate is 100 g/m²/24 h. The indentations are filled with Formulation D.

### Embodiments 33 to 36

The structural parameters of the cover film combination used in the patch as defined in Embodiments 33 to 36 are as follows, and others are the same as those in embodiment 32. The indentations are filled with Formulation D.

| Index | | Embodiment 33 | Embodiment 34 | Embodiment 35 | Embodiment 36 |
|---|---|---|---|---|---|
| Cover film | MVTR (g/m²/24 h) | 400 | 4,000 | 7,000 | 10,000 |
| | Thickness/mm | 0.2 | 2 | 0.5 | 10 |
| Indentation | Depth/mm | 0.2 | 2 | 0.5 | 10 |
| | Opening width/mm | 2 | 20 | 3 | 40 |
| | Opening length/cm | 2 | 20 | 10 | 50 |
| The proportion of opening area in the total area of contact surface/% | | 70 | 75 | 80 | 95 |
| Barrier film | MVTR (g/m²/24 h) | 400 | 4,000 | 7,000 | 10,000 |

### Embodiment 37

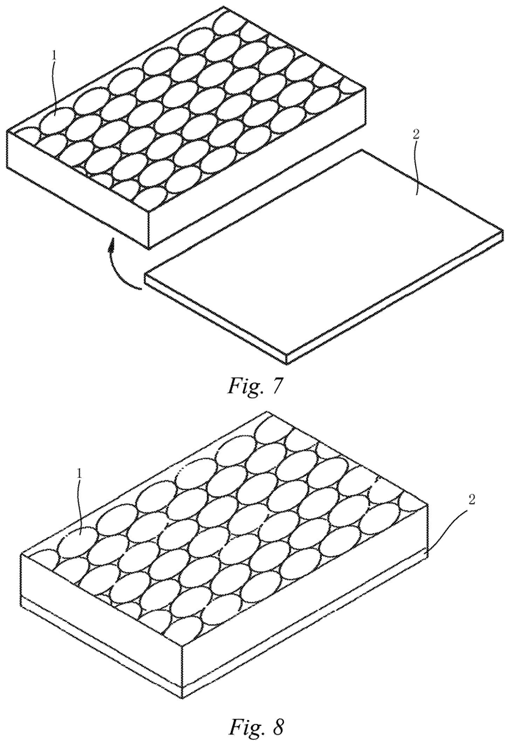

A patch, which comprises the cover film as shown in Figure 7 and Formulation D. The cover film is a polyurethane film, of which the moisture vapor transmission rate is 100 g/m²/24 h, the thickness is 3 millimeters, the length is 10 centimeters, and the area is 15 square centimeters. The contact surface of the cover film to the skin is provided with several indentations 1. The indentation is configured as a hemispheroid, and the height of the hemispheroid is perpendicular to the contact surface. The indentations are arranged uniformly, the number of indentations per square centimeter of the covering film is 0.8, each hemispheroidal indentation is 3 millimeters high, the opening area of the indentation 1 accounts for 70% of the total area of the contact surface, and the outer edges of the bottoms of two neighboring hemispheroids are tangent to each other. One side of the indentation 1 away from the opening is also attached with a barrier film 2 so as to form the covering combination as shown in Figure 8. The barrier film 2 is a polyurethane film, of which the moisture vapor transmission rate is 100 g/m²/24 h. The indentations are filled with Formulation D.

### Embodiments 38 to 41

The structural parameters of the cover film combination used in the patch as defined in Embodiments 38 to 41 are as follows, and others are the same as those in embodiment 37. The indentations are filled with Formulation D.

| Index | | Embodiment 38 | Embodiment 39 | Embodiment 40 | Embodiment 41 |
|---|---|---|---|---|---|
| Cover film | MVTR (g/m²/24 h) | 400 | 4,000 | 7,000 | 10,000 |
| | Thickness/mm | 0.2 | 2 | 0.5 | 10 |
| | Length/cm | 3 | 30 | 8 | 50 |
| | Area/cm² | 5 | 500 | 10 | 1,000 |
| Indentation | Number of indentations per square centimeter | 0.2 | 20 | 0.5 | 100 |
| | Depth/mm | 0.2 | 2 | 0.5 | 10 |
| The proportion of opening area in the total area of contact surface/% | | 70 | 75 | 80 | 95 |
| Barrier film | MVTR (g/m²/24 h) | 400 | 4,000 | 7,000 | 10,000 |

### Embodiment 42

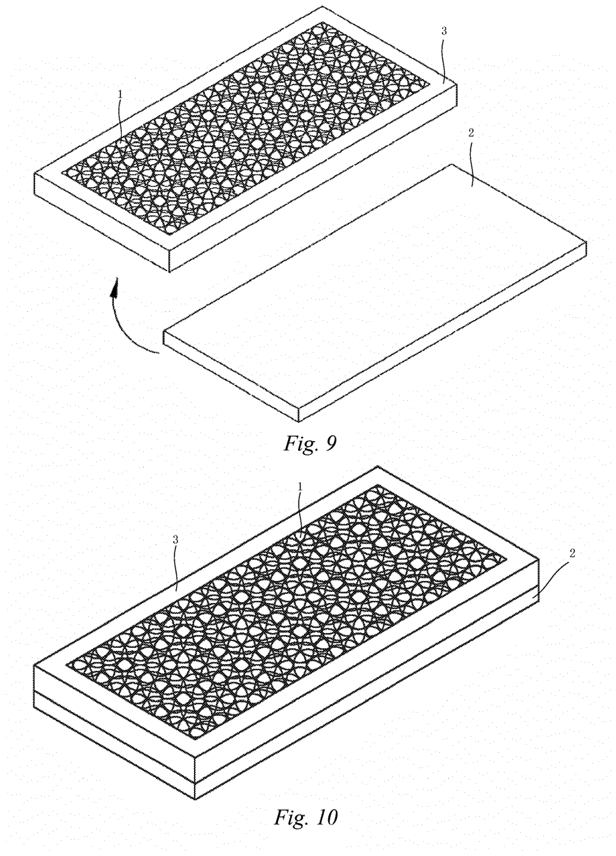

A patch, which comprises the cover film as shown in Figure 9 and Formulation D. The cover film is a polyurethane film, of which the moisture vapor transmission rate is 100 g/m²/24 h, the thickness is 3 millimeters, the length is 10 centimeters, and the area is 15 square centimeters. The cover film is provided with only one indentation 1, which has a cavity and a bonding layer 3 surrounding the cavity. The cavity is 3 millimeters deep, the cavity is provided with the first surface in contact with the skin, the first surface is provided with several first through-holes, and the opening area of the first through-holes accounts for 70% of the total area of the first surface. The indentation cavity comprises one or more layers of drug storage cavity units, each layer of the drug storage cavity units comprises several drug storage micro-cavities, each of the drug storage micro-cavities is provided with a second through-hole on the side wall (not marked in the figure), and the second through-hole is used for connecting the two neighboring drug storage micro-cavities. All the drug storage micro-cavities form a loofah-like network structure together. One side of the cover film away from the first through-hole is also attached with a barrier film 2 so as to form the cover combination as shown in Figure 10. The barrier film 2 is a polyurethane film, of which the moisture vapor transmission rate is 100 g/m²/24 h. The indentations are filled with Formulation D.

### Embodiment 43

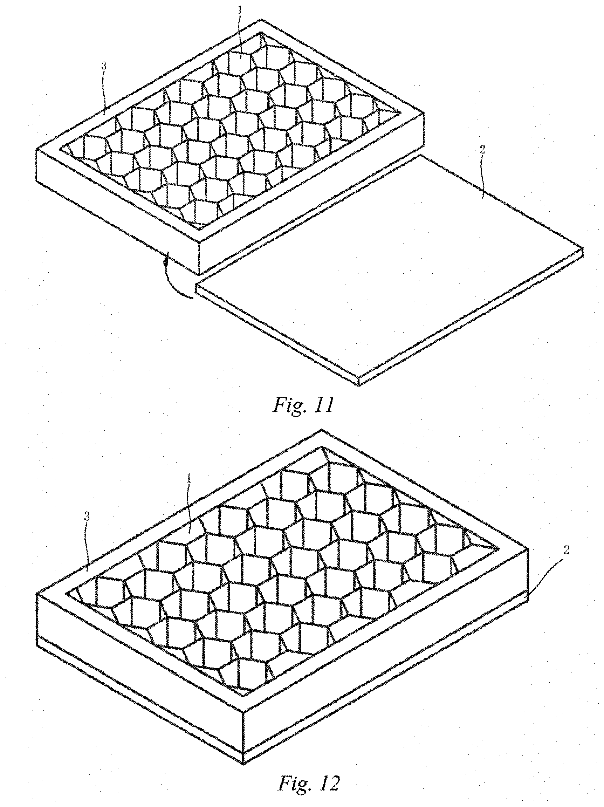

The cover film as shown in Figure 11 is a polyurethane film, of which the moisture vapor transmission rate is 400 g/m²/24 h, the thickness is 0.2 millimeters, the length is 3 centimeters, and the area is 5 square centimeters. The cover film is provided with only one indentation 1, which has an indentation cavity and a bonding layer 3 surrounding the indentation cavity. The indentation cavity is 0.2 millimeters deep, the indentation cavity is provided with the first surface in contact with the skin, the first surface is provided with several first through-holes, and the opening area of the first through-holes accounts for 70% of the total area of the first surface. One or more layers of drug storage cavity units are provided in the indentation cavity, each layer of the drug storage cavity units comprises several drug storage micro-cavities, each of the drug storage micro-cavities is provided with a second through-hole on the side wall (not marked in the figure), and the second through-hole is used for communication of the two neighboring drug storage micro-cavities. The drug storage micro-cavity is a regular-prism-shaped cavity, and the drug storage micro-cavities are arranged in a honeycomb structure. The drug storage micro-cavities of the two neighboring layers are also in a staggered arrangement. One side of the cover film away from the first through-hole is also attached with a barrier film 2 so as to form the cover combination as shown in Figure 12. The barrier film 2 is a polyurethane film, of which the moisture vapor transmission rate is 400g/m²/24 h. The indentations are filled with Formulation D.

### Embodiments 44 to 46

The structural parameters of the cover film combination used in the patch as defined in Embodiments 44 to 46 are as follows, and others are the same as those in embodiment 43. The indentations are filled with Formulation D.

| Index | | Embodiment 44 | Embodiment 45 | Embodiment 46 |
|---|---|---|---|---|
| Cover film | MVTR (g/m²/24 h) | 4,000 | 7,000 | 10,000 |
| | Thickness/mm | 2 | 0.5 | 10 |
| | Length/cm | 30 | 8 | 50 |
| | Area/cm² | 500 | 10 | 1,000 |
| Drug storage cavity | Depth/mm | 2 | 0.5 | 10 |
| The proportion of the opening area in the total area of the first surface/% | | 75 | 80 | 95 |
| Barrier film | MVTR (g/m²/24 h) | 4,000 | 7,000 | 10,000 |

### Effect embodiment 1

A layer of Formulation A, 2 millimeters thick, was applied onto a piece of non-woven fabric, and a part of Formulation A was absorbed into the non-woven fabric. This piece of non-woven fabric absorbed with Formulation A was placed on the upper arm skin of a human subject, and covered by a layer of impermeable plastic film. The plastic film was fixed by an adhesive film on the skin. The skin covered by Formulation A was anesthetized after about 90 minutes. Formulation A was removed from the skin 24 hours after the start of medication. The skin covered by Formulation A was in the anesthetized state when Formulation A was removed and stayed anesthetized for one hour following the removal of Formulation A.

### Effect embodiment 2

A patient had suffered postherpetic neuralgia on the back skin for more than 6 years. A layer of Formulation B, about 1 millimeter thick, was applied onto the affected skin, and covered by a layer of impermeable plastic film. The plastic film was fixed by an adhesive film on the skin. The purpose of the plastic film was to prevent the evaporation of the moisture in Formulation. After about 90 min, this patient's pain began to be relieved significantly. The formulation and the plastic film were maintained on this patient's skin for 18 hours, and then removed. This patient felt almost no pain for approximately 20 hours: 90 minutes after the start of the medication and several hours after termination of the medication. This very satisfactory analgesia was better than that of any drug she had used in the past six years, and helped her enjoy a good sleep. In the following months, she repeatedly used Formulation B and other formulations of embodiments, including Formulation C, in the same manner, with similar satisfactory results.

### Effect embodiment 3

A patient suffered herpes zoster on 18 × 18 centimeters leg skin and the skin of two toes, and this patient was in the herpes period of herpes zoster. The affected skin was sore and swollen. Formulation C, about 1 millimeter thick, was applied onto the affected skin, and then covered by a layer of plastic film, in order to prevent the evaporation of the moisture in the formulation. The formulation and the plastic film were maintained on this patient's skin for 25 hours. Except for the first hour, the patient felt little pain during the 25 hours. After the formulation was removed, the redness and swelling of the affected skin were greatly alleviated. This patient used Formulation C in a similar manner 4 hours later. The thickness of Formulation C layer on the skin was about 2 millimeters this time. This patient removed the formulation and the plastic film after 35 hours. Except for the first hour, this patient got a very satisfactory analgesia during the 35 hours.

### Effect embodiment 4

A patient's entire right leg and partial pelvis were excised for osteocarcinoma. After the excision, she felt an intense pain on the skin at the incision (the incision was covered by this patient's own skin saved during the operation). The physicians considered that her pain was caused by the neurologic impairment from the operation. In addition, she also felt an acute phantom limb pain (a pain that amputees often feel in a limb that no longer exists) on the right leg there was already removed. To alleviate the two pains of this patient, Formulation C, about 1 millimeter thick, was applied by the medical staff onto the affected skin, and covered by a layer of plastic film with adhesive on the sides. One hour later, her skin pain and phantom limb pain basically went away. She maintained the formulation on the skin for 24 hours. The effective analgesia was maintained for about 24 hours after the removal of the formulation. This patient repeated the medication every two days in the following weeks. The analgesia was so good that she didn't need to take analgesics orally most of the time. It was an unexpected finding that Formulation C was effective in easing the phantom limb pain.

### Effect embodiment 5

A patient had suffered serious postherpetic neuralgia on the skin of back (30 × 30 centimeters) and the skin of chest (15 × 15 centimeters) for about 8 years. None of the treatments received worked. Formulation C, about 2 millimeters thick, was applied onto the affected skin, and covered by a layer of plastic film. After about 1 hour, this patient's pain began to reduce significantly. The formulation and the plastic film were maintained on this patient's skin for 36 hours before removal. Except for the first hour, the analgesia was very satisfactory during the 36 hours.

### Effect embodiment 6

To relieve the caesarean incision pain of a patient, a layer of Formulation D, 3 millimeters thick and 1.5 centimeters wide, is applied by the medical staff onto the sutured incision, and then covered by the covering combination as shown in Figure 2. The lidocaine molecules in the formulation immediately begin to penetrate the incision and the surrounding tissues. This patient's surgical incision pain begins to reduce significantly within 15 minutes. The satisfactory analgesia lasts for more than 12 hours. When this patient feel the pain again, Formulation D and the covering combination are removed by the medical staff, and then fresh Formulation D and covering combination are used once more in the same manner. Afterwards, when this patient needs analgesia, the medical staff use Formulation D and the covering combination in the same manner.

### Effect embodiment 7

To relieve the surgical incision pain of a patient receiving cholecystectomy, a layer of Formulation F, 3 millimeters thick, was applied by the medical staff onto the sutured incision, and then covered by the covering combination as shown in Figure 4. The bupivacaine molecules in the formulation immediately begin to penetrate the incision and the surrounding tissues. This patient's surgical incision pain begins to reduce significantly within 15 minutes. The satisfactory analgesia lasts for more than 12 hours. Afterwards, when this patient needs analgesia, the medical staff use Formulation F and the covering combination in the same manner.

### Effect embodiment 8

To relieve the surgical incision pain of a patient after an appendicitis surgery, a layer of Formulation G, 3 millimeters thick, was applied by the medical staff onto the sutured incision, and then covered by a layer of medical film. The tetracaine molecules in the formulation immediately begin to penetrate into the incision and the surrounding tissues. This patient's surgical incision pain begins to reduce significantly within 10 minutes. The satisfactory analgesia lasted for more than 24 hours. Afterwards, when this patient needs analgesia, the medical staff use Formulation G and the medical film in the same manner.

### Effect embodiment 9

A patient suffered the pain of periarthritis on both shoulders. A layer of Formulation C, 1-2 millimeters thick, is applied onto the skin of this patient's two shoulders, and then is covered by the covering combination as shown in Figure 6. After one hour, this patient's shoulder pain almost disappears. This satisfactory analgesia lasts for 8 hours on one shoulder, and more than 18 hours on the other shoulder.

### Effect embodiment 10

A layer of Formulation A is applied onto a layer of gauze covering the burn wound of a person. The formulation permeates into the wound via the gauze, and lidocaine is delivered to the wound tissues, thus relieving the wound pain greatly. Because of the slow release function of the formulation, the analgesia lasts for many hours. Afterwards, when this patient needs analgesia, the formulation layer is directly applied onto the burn wound, and then covered by the cover film as shown in Figure 6, so the evaporation rate of the moisture in the formulation is reduced, thereby extending the effective analgesia duration.

As for the pain arising from the removal of burn scabs on the wound, the covering film as shown in Figure 6 is covered on the formulation layer already applied onto the gauze layer, so the evaporation rate of the moisture in the formulation is reduced, thereby extending the effective analgesia duration.

### Effect embodiment 11

A patient suffers from the pain of osteoarthritis on a knee. A layer of Formulation C, about 2 millimeters thick, is applied onto the entire knee skin, and then covered by the covering combination as shown in Figure 6. This patient maintains the formulation on the knee for 18 hours before removing it, and repeats the aforesaid procedures daily. This patient's pain is greatly reduced.

### Effect embodiment 12

A patient suffers from the pain caused by gout on the ankle. A layer of Formulation C, about 2 millimeters thick, is applied onto the entire ankle skin, and then covered by the covering combination as shown in Figure 6. This patient maintains the formulation on the ankle for 18 hours before removing it, and repeats the aforesaid procedures daily. This patient's pain is greatly reduced.

### Effect embodiment 13

A patient suffers from back pain. A layer of Formulation C, about 2 millimeters thick, is applied onto the skin over the pain, and then covered by the covering combination as shown in Figure 6. This patient maintains the formulation on the back skin for 18 hours before removing it, and repeats the aforesaid procedures daily. This patient's pain is greatly reduced.

### Effect embodiment 14

A 2 millimeters thick layer of any of the pharmaceutical compositions as described in Embodiments 8 through 26, is applied onto a piece of non-woven fabric, and a part of the formulation is absorbed into the non-woven fabric. This piece of non-woven fabric absorbed with the formulation is placed on the upper arm skin of a human subject, and covered by a layer of impermeable plastic film. The plastic film is fixed by an adhesive film on the skin. The skin covered by the formulation is anesthetized in about 90 min. The formulation is removed from the skin 24 hours after the start of medication. The skin covered by the formulation is in the anesthetized state when the formulation is removed and one hour after removal of the same.

### Effect embodiment 15

To relieve the caesarean incision pain of a patient, a patch as defined in any of the Embodiments 27 through 46 is applied by the medical staff onto the sutured incision. The lidocaine molecules in the formulation immediately begin to penetrate into the incision and the surrounding tissues. This patient's surgical incision pain begins to reduce significantly within 15 minutes. During the analgesia treatment, this patient often has the routine movements such as turning over and lying on her side, and the covering film is resistant to the external squeezing force, preventing the formulation layer from being squeezed out, thereby maintaining the thickness of the formulation layer. The satisfactory analgesia lasts for more than 12 hours. Afterwards, when this patient needs analgesia again, the medical staff use Formulation D and the covering combination in the same manner. During the analgesia treatment, the covering film is resistant to the external squeezing force, preventing the formulation layer from being squeezed out, thereby maintaining the thickness of the formulation layer.

### Effect embodiment 16

Any of the pharmaceutical composition as defined in Embodiments 1 through 26, when used for treating the pain in the herpes period of herpes zoster, postherpetic neuralgia, neuroma pain, phantom limb pain, diabetic peripheral neuropathic pain, arthralgia, osteoarthritis pain, back pain, pain caused by gout, pain caused by soft tissue injury, postoperative incision pain, burn pain or pain caused by removal of burn scabs, achieves continuous analgesia for 12 to 30 hours.

Although the specific embodiments of the present invention have been described above, it will be understood by those skilled in the art that these are merely illustrative, and that various alterations or modifications can be made to these embodiments without departing from the principle and essence of the present invention. Therefore, the scope of protection of the present invention is defined by the appended claims.

## Claims

1. A pharmaceutical composition, wherein the pharmaceutical composition comprises a single anesthetic active ingredient which comprises a part of the anesthetic active ingredient dissolved in water and another part of the anesthetic active ingredient existing in an undissolved form; the mass percent content of the anesthetic active ingredient in the pharmaceutical composition is 2% or more; the mass percent content of water-dissolved anesthetic active ingredient in the pharmaceutical composition is 1% or less; and the anesthetic active ingredient comprises lidocaine, tetracaine, bupivacaine, articaine, cinchocaine, dibucaine, etidocaine, levobupivacaine, mepivacaine, prilocaine, ropivacaine, trimecaine, benzocaine or procaine.

2. The pharmaceutical composition according to claim 1, wherein the pH value of the pharmaceutical composition is higher than 7, preferably higher than 8, and more preferably 7-13, 7-11 or 7.5-9.5;
wherein, preferably, an alkaline substance is used for regulating the pH value of the pharmaceutical composition to higher than 7, higher than 8, 7-13, 7-11 or 7.5-9.5, and the alkaline substance is one or more of sodium tripolyphosphate, sodium bicarbonate, sodium hydroxide, and potassium hydroxide; more preferably, the alkaline substance is sodium hydroxide and/or potassium hydroxide; and further more preferably, the alkaline substance is sodium hydroxide.

3. The pharmaceutical composition according to claim 1 or 2, wherein the mass percent content of the anesthetic active ingredient in the pharmaceutical composition is more than 3%, more than 4%, more than 5%, more than 8% or more than 10%; preferably 3%-15%, 4%-10% or 5%-8%;
and/or, the mass percent content of the water-dissolved anesthetic active ingredient in the pharmaceutical composition is less than 0.7%, 0.5%-0.7% or less than 0.5%;
and/or, among the particles of the anesthetic active ingredient, the single particle weight of more than 80% of the particles of anesthetic active ingredient is in the range of 0.01-10 mg.

4. The pharmaceutical composition according to at least one of claims 1 through 3, wherein the pharmaceutical composition further comprises a suspension agent; wherein the suspension agent is preferably carbomer, and the model of the suspension agent is preferably Pemulen TR-2; the mass percent content of the suspension agent in the pharmaceutical composition is preferably 0.10%-0.50%;
and/or, the pharmaceutical composition further comprises a thickener; wherein the thickener is preferably one or more of starch, carbomer and cellulose, and more preferably one or more of starch, hydroxyethyl cellulose and hydroxypropyl cellulose; the mass percent content of the thickener in the pharmaceutical composition is preferably 0.02%-4%, more preferably 0.02%-2%, and further more preferably 0.02%-1 %;
and/or, the pharmaceutical composition further comprises glycerin and/or propylene glycol, and the mass percent content of the glycerin and/or propylene glycol in the pharmaceutical composition is preferably 2%-25%.

5. The pharmaceutical composition according to at least one of claims 1 through 4, wherein when the anesthetic active ingredient is tetracaine, the mass percent content of the tetracaine in the pharmaceutical composition is more than 0.5%, and the mass percent content of the water-dissolved tetracaine in the pharmaceutical composition is less than 0.1%.

6. A manufacturing method for the pharmaceutical composition according to any one of claims 1 to 5, wherein the manufacturing method comprises the following step of: mixing the different ingredients of the pharmaceutical composition;
preferably the manufacturing method comprises the following steps of: (1) mixing all ingredients except for the anesthetic active ingredient in the pharmaceutical composition, and obtaining a mixed solution; (2) heating the mixed solution and the anesthetic active ingredient, and then cooling to the room temperature;
in the step (2), the heating temperature is preferably 75°C-85°C; and
in the step (2), the heating is conducted preferably with stirring.

7. A patch, comprising a cover film and the pharmaceutical composition according to any one of claims 1 to 5, wherein the contact surface of the cover film to the skin is provided with at least one indentation, the opening area of the indentation accounts for more than 70% (100% excluded) of the total area of the contact surface, and the indentation is filled with the pharmaceutical composition.

8. The patch according to claim 7, wherein the thickness of the cover film is 1-20 millimeters, and preferably 3-10 millimeters; the length of the cover film is 3-50 centimeters, and preferably 8-30 centimeters; the area of the cover film is 5-1,000 square centimeters, and preferably 10-500 square centimeters;
and/or, the indentation is configured as one or more of a cuboid, a cylinder and a hemispheroid; wherein, preferably, the axis of the cylinder is perpendicular to the contact surface, and the axis of the hemispheroid is perpendicular to the contact surface; more preferably the indentation is configured as a hemispheroid, and the opening of each indentation is tangent to the opening of the adjacent indentation;
and/or, the depth of the indentation is less than 10 millimeters, preferably 0.2-10 millimeters, and more preferably 0.5-3 millimeters.

9. The patch according to claim 7 or claim 8, wherein the cover film is provided with a indentation, the indentation has a cavity, and the opening width of the indentation is 2-40 millimeters, and preferably 3-20 millimeters; the opening length of the indentation is 2-50 centimeters, and preferably 5-20 centimeters;
or, the cover film is provided with two or more indentations, and the number of the indentations per square centimeter of the cover film is 0.2-100, and preferably 0.5-20; the spacing between the two neighboring indentations is 0-5 millimeters, and preferably 0-2 millimeters; wherein, preferably, the two neighboring indentations are not connected;
or, the cover film is provided with only one indentation, the indentation has an indentation cavity, the contact surface of the indentation cavity to the skin is provided with several first through-holes, one or more layers of drug storage cavity units are provided in the indentation cavity, each layer of the drug storage cavity units comprises several drug storage micro-cavities, a side wall of each of the drug storage micro-cavities is provided with a second through-hole, and the second through-hole is used for communication of the two neighboring drug storage micro-cavities; the opening area of the first through-hole accounts for more than 70% (100% excluded) of the total area of the first surface;
wherein, preferably, all the drug storage micro-cavities form a loofah-like network structure together;
wherein, preferably, the drug storage micro-cavity is a regular-prism-shaped cavity, and the drug storage micro-cavities are arranged in a honeycomb structure;
wherein, preferably, the drug storage micro-cavities of the two neighboring layers are in a staggered arrangement; and
wherein, preferably, other surfaces of the indentation cavity except for the first surface are all of a sealed structure.

10. The patch according to at least one of claims 7 to 9, wherein the depth of the indentation is equal to the thickness of the covering film; wherein one side of the indentation away from the opening is partially or totally attached with a barrier film;
and/or, the indentations have the same structure, and the indentations are arranged uniformly or in an array;
and/or, the cover film is a foam plastic film, and preferably a polyurethane film; and wherein the moisture vapor transmission rate of the polyurethane film is preferably 100-10,000 g/m²/24 h, and more preferably 400-4,000 g/m²/24 h.

11. An application of the pharmaceutical composition according to any one of claims 1 to 5 in the manufacturing of a drug for treating the pain in the herpes period of herpes zoster, postherpetic neuralgia, neuroma pain, phantom limb pain, diabetic peripheral neuropathic pain, arthralgia, osteoarthritis pain, back pain, pain caused by gout, pain caused by soft tissue injury, postoperative incision pain, burn pain or pain caused by removal of burn scabs.

12. An application of the patch according to any one of claims 7 to 10 in the manufacturing of a medical device for treating the pain in the herpes period of herpes zoster, postherpetic neuralgia, neuroma pain, phantom limb pain, diabetic peripheral neuropathic pain, arthralgia, osteoarthritis pain, back pain, pain caused by gout, pain caused by soft tissue injury, postoperative incision pain, burn pain or pain caused by removal of burn scabs.

13. An analgesic method of using the pharmaceutical composition according to any one of claims 1 to 5, wherein the analgesic method comprises the following steps of: applying the pharmaceutical composition onto the affected skin, and then covering the pharmaceutical composition with a barrier film;
wherein the affected skin is preferably the skin suffering from or over the pain in the herpes period of herpes zoster, postherpetic neuralgia, neuroma pain, phantom limb pain, diabetic peripheral neuropathic pain, arthralgia, osteoarthritis pain, back pain, pain caused by gout, pain caused by soft tissue injury, postoperative incision pain, burn pain or pain caused by removal of burn scabs;
wherein preferably said pharmaceutical composition is maintained on said skin for more than 8, 12, 18, 24, or 30 hours; and
wherein the thickness of the pharmaceutical composition on the affected skin is preferably not less than 0.5, 1 or 2 millimeters.

14. An analgesic method by using the patch according to any one of claims 7 to 10, wherein the analgesic method comprises the following step of: bringing the opening of the indentation in the patch into contact with the affected skin;
wherein the affected skin is preferably the skin suffering from or over the pain in the herpes period of herpes zoster, postherpetic neuralgia, neuroma pain, phantom limb pain, diabetic peripheral neuropathic pain, arthralgia, osteoarthritis pain, back pain, pain caused by gout, pain caused by soft tissue injury, postoperative incision pain, burn pain or pain caused by removal of burn scabs;
wherein preferably the the patch is maintained on the affected skin for more than 8, 12, 18, 24, or 30 hours; and
wherein the thickness of the pharmaceutical composition contained in the patch on the affected skin is preferably kept not less than 0.5, 1 or 2 millimeters.
